Europäisches Patentamt

(19) European Patent Office

Office européen des brevets

(11) Publication number: **0 162 545**
**A2**

(12) # EUROPEAN PATENT APPLICATION

(21) Application number: **85301958.6**

(22) Date of filing: **21.03.85**

(51) Int. Cl.⁴: **C 12 N 1/38**
**//C12P7/06, C02F3/00**

(30) Priority: **23.05.84 US 612985**

(43) Date of publication of application:
**27.11.85 Bulletin 85/48**

(84) Designated Contracting States:
**BE DE FR GB IT NL**

(71) Applicant: **BETZ EUROPE, INC.**
**4636 Somerton Road**
**Trevose Pennsylvania 19047(US)**

(72) Inventor: **Whitekettle, Wilson Kurt**
**215 Hampton Hall Lane**
**Conroe Texas 77302(US)**

(72) Inventor: **Friend, Patric Lee**
**609 Glen Haven St.**
**Conroe Texas 77385(US)**

(72) Inventor: **Weaver, Timothy Wayne**
**101 E Lakeview Terrace**
**Montgomery Texas 77356(US)**

(74) Representative: **Gore, Peter Manson et al,**
**W.P. THOMPSON & CO. Coopers Building Church Street**
**Liverpool L1 3AB(GB)**

(54) Enhanced bioconversion processes.

(57) The present invention is directed to methods and substances which can be utilized to enhance bioconversion process. It relates to a method of increasing the capacity and rate of a microbial cell population's bioconversion of a substrate in an aqueous system, without an attendant significant increase in (i) the microbial cell population and (ii) solid waste production from said bioconversion, which microbial cells maintain their life cycle and reproduce through the production, use and storage of energy and the maintenance of an energy reservoir, characterized in that there is added to said system an effective amount of a substance or substances which will effectively impede the natural tendency of the microbial cell during its bioconversion of said substrate to attain its normal energy reservoir level and will thereby promote greater bioconversion of the substrate because of the microbial cell's attempt to achieve its normal energy reservoir. It also relates to a composition characterized by a member selected from

(A) beta-bromo-beta-nitrostyrene, and bis-(trichloromethyl) sulfone and at least one member selected from
(B) 4,7 diphenyl-1, 10 phenathroline;
2-mercaptobenzothiazole;
myristic acid;
N,N,N',N' tetramethyl p-phenylene diamine;
rotenone;
salicylanilide;
tetraphenylboron; thiosalicylic acid; and
dicyclohexylcarbodiimide.

-1-

DESCRIPTION

ENHANCED BIOCONVERSION PROCESSES

The present invention is directed to any system or systems where microbial populations are specifically utilized for a given purpose. The invention has as its objective the enhancement of these systems to achieve the purpose or purposes with greater efficacy and with minimization of some of the side problems experienced prior to the advent of the present invention.

Bioconversion is a process wherein living organisms are employed to carry out a physical and/or chemical change in a substance. The substance can be natural or man-made. The bioconversion processes to which the present invention is directed include both synthetic and degradative processes. Examples of bioconversion processes include, but are not limited to, fermentations, wastewater treatment, biopolymer production, biomining, and microbially-enhanced oil recovery. Fermentations in this context are defined as the process of microbial production of useful products which are primary or secondary metabolites of microbial origin, such as antibiotics, ethanol, acetone, glycols, organic acids, amino acids, vitamins, antimetabolites, esters, ethers, ketones, aldehydes, flavorings, foods, hormones, enzymes, lipids, alkaloids, surfactants, and gases such as methane. The microbial production process may be either

-2-

aerobic or anaerobic.

Wastewater treatment is defined as the process by which microbial populations are used to remove and degrade waste substances, generated either by industrial activity or by municipalities, contained in an aqueous environment. This can be achieved by trickling filters, activated sludge systems, and anaerobic treatment systems.

Biopolymer production is the conversion by a microbial population of substrates to homopolymers or heteropolymers having desired properties. Biomining is the extraction and concentration of mineral elements from ores and rocks, mediated by microorganisms.

Microbial enhanced oil recovery is the process of freeing oil deposits trapped in geologic formations, both subterranean and surface, through the action of microorganisms deliberately introduced into the geologic formation. The microorganisms employed are selected for their ability to reduce the viscosity of the trapped oil or to increase dramatically the pressure in the formation, to aid in forcing out the trapped oil.

As indicated above, the use of microbial consumption of a substrate(s) for various purposes has been widespread. Because of the technical and commercial success of these applications, whether for waste water treatment or for the production of metabolites through fermentation and the like procedures, the respective industries are continually researching and developing different techniques, microbial populations, substrates, etc. in an attempt to further enhance these applications from both effectiveness and economic points of view.

It has now been found possible to increase the overall effectiveness of microbial populations' consumption and/or bioconversion of an organic substrate, without an attendant increase in (i) the microbial population itself, and (ii) solid waste production (including but not limited to both sludge and biomass).

With respect to waste water treatment, the process objective is to remove as much waste as possible from the water being treated while producing the least amount of biomass or sludge, since such requires disposal which, as is clearly evident, is expensive both from an effort and cost point of view.

Likewise, with the production of metabolites (useful chemicals), it is desirable to achieve as close to total consumption or bioconversion of the substrate as possible, as quickly as possible, with the use of smaller microbial populations (i.e., have energies expended for bioconversion rather than in the reproduction of additional cells) in order to positively effect the economies of the production process. It is common in systems as described above for a good portion of the cell's energies to be expended in the production of new microbial cells which, if not successful in competing for survival with the other cells, die and thus add to the biomass that must be disposed.

More specifically, one can view cellular activities in terms of "bio-economics". In order to obtain maximal proliferation, or at the least subsistence, a cell must maximize its efficiencies

0162545

-4-

so that there is insurance of a niche for its continuing activities.

To maximize its efficiencies, all activities of the cell are intercontrolled and intercommunication is carried out on the molecular level.

Maximum efficiency is selected for daily by competition from other species and environmental pressures. If activities are not optimized the price the specie must pay is extinction.

Cellular economics are based upon energy expenditures. These transactions must be executed with as little loss as possible so that energy can be laid in reserve for other coordinate transactions. The currency utilized by the cell is the compound ATP. Accordingly, the ultimate control on cellular activities is governed by the intracellular level of ATP. If the needed level is depleted more substrate is processed through the cell to bring activities up to their functional level. If the needed level of ATP cannot be restored the cell accumulates a loss and ground is lost in terms of proliferation. This loss is ultimately reflected in the total amount of biomass which is produced under a specific set of conditions - one measurement of the cell's proliferation.

If a recurrent loss is seen the cell will eventually burn-up its "saved" or "stored" energy and, once this is depleted, death ensues. For a more comprehensive description of the "ATP" phosphorylation phenomena, the following can be considered: Bioenergetics, A. Lehninger, 2nd Edition, pp. 90 - 92 and Scientific American, "How cells make ATP" March, 1978, pp. 104 - 122.

0162545

-5-

It has been found that exposure of microbial cell populations to certain chemicals referred to as "uncouplers" places the cells in a stressed state whereby ATP production is made less efficient. At low levels the cell can still proliferate, although not as competently as in a "clean" system, by processing more substrate and/or producing less biomass. At increasingly higher levels, effects upon the cell are to eventually break down its economic system until cellular bankruptcy and death occur. This chemical induction of energy uncoupling can be exploited in a variety of systems.

As indicated hereinabove, in biological waste treatment the process objective is to remove as much waste (pollutant) from the polluted influent as possible while producing the least amount of biomass or sludge for final disposal. Uncouplers would chemically permit this by lowering energy levels in the metabolizing activated sludge population and coordinately causing them to take up more wastes and produce less biomass.

In fermentation systems uncoupler dosing is effective because it increases the rate of the process, e.q., the rate of ethanol production from glucose, while concurrently lowering energy costs for separation of the cellular fraction.

According to the present invention there is provided a method of increasing the capacity and rate of a microbial cell population's bioconversion of a substrate in an aqueous system, without an attendant significant increase in (i) the microbial cell population and (ii) solid waste production from said bio conversion, which microbial cells maintain their life cycle and reproduce through the production, use and storage of energy and the maintenance of an energy reservoir, characterized in that there is added to said system an effective amount of a substance or substances which will effectively impede the natural tendency of the microbial cell during its bioconversion of said substrate to attain its normal energy reservoir level and will thereby promote greater bioconversion of the substrate because of the microbial cell's attempt to achieve its normal energy reservoir.

The substance or substances are desirably such as to uncouple the microbial cells' energy production from substrate bioconversion by effectively blocking to the necessary degree energy transfer to the energy reservoir of the cells. The amount of substance or substances used is preferably such as to not totally block the transfer of the energy produced to the energy reservoir of the cells and thereby permit the cells to continue to live and bioconvert the substrate.

The substance or substances preferably comprise an oxidative uncoupler or uncouplers, such as, for example, a nitrostyrene compound, (preferably beta-bromo-beta-nitrostyrene).

Examples of the substance or substances are bis-(trichloromethyl)sulfone; 4, 7 diphenyl-1, 10 phenathroline; 2-mercaptobenzothiazole; myristic acid; N,N,N',N' tetramethyl p-phenylene diamine; rotenone; salicylanilide; tetraphenylboron; thiosalicyclic acid; dicyclohexylcarbodiimide; beta-bromo-beta-nitrostyrene; and mixtures thereof.

The substrate may be an organic substrate, for example a carbohydrate producing ethanol. A preferred aqueous system is a waste water system.

According to one embodiment of the present invention there is provided a composition characterized by a member selected from:

(A) beta-bromo-beta-nitrostyrene, and bis-(trichloromethyl)sulfone and at least one member selected from

(B) 4, 7 diphenyl-1, 10 phenathroline;
2-mercaptobenzothiazole;
myristic acid
N,N,N',N' tetramethyl p-phenylene diamine;
rotenone;
salicylanilide;
tetraphenylboron; thiosalicylic acid; and dicyclohexylcarbodiimide.

Utilization of these levels permits the cells to continue to live and therefore bioconvert the organic substrate, at a more rapid rate. In these systems the energy which is transferred produces, and is stored in the cell(s) as adenosine triphosphate (ATP).

The foregoing methods are applicable in any system, and in particular to the waste water treatment and fermentation systems which are dependent upon microbial activity. As apparent, the source of the substrate consumed by the cells will be different depending upon the system utilized. In waste treatment systems, the organic substrate or source may be organic waste products, remains of deceased microbial cells, and/or the excretion of other living microbial cells.

In a fermentation process where metabolites (for example, alcohols such as ethanol, butanol, glycerol, etc; butylene glycol, etc.; organic acids such as acetic, lactic, formic, propionic, glutamic, citric, etc.; organic compounds such as methane, ethane and the like; and products such as acetone and hydrogen) are produced, the carbon source may be chosen appropriately (glucose, molasses, cellulose, chitin, starch, proteins, proteoses, peptides, oils, fats, lipids, saccharides, alcohols, aldehydes, etc.) as with the particular species of microbial cells being used. Metabolites such as these possess commercial utility as food products or additives, as beverage additives, as energy sources, and as feed stocks for chemical synthesis.

More specifically, for example, the production of ethanol is a time-honored process most commonly associated with the fermentation industry. Ethanol is a major primary metabolite produced by many microorganisms fermenting many varied substrates, but has had primary use as a beverage, for its cost of production by fermentation was not competitive with its cost of production by chemical conversion of petroleum feedstocks (chiefly ethylene). Recent price increases in petroleum feedstocks have made bioconversion of substrates to ethanol more competitive. Coupled with the proposed use of ethanol as an automobile fuel (gasohol), a large market for bioproduced ethanol is emerging. Enhancement of ethanol production by uncouplers, with no alterations in current processes or equipment, would offer an increased competitive edge for the bioproduction of ethanol.

Microbial production of any compound, however, suffers from two problems which can be alleviated by treatment with an uncoupler. Increased production of the endproduct (ethanol) is always desirable, the goal being rapid production with 100% efficiency of theoretical substrate conversion to product formation. This goal is not always achieved, due to a varietyof natural microbial control mechanisms which are frequently beyond influence by the manufacturer. Some influence is attempted by varying the physiochemical environment of the microbes or by genetic or physiological manipulation of the microbes, with varying degrees of success. Another problem results from attempts to separate the proliferating biomass from the product, which may become proportionally more difficult as the biomass increases. A variety of approaches is used for product (ethanol) separation, such as physicochemical changes of the environment or mechanical means.

Treatment with an uncoupler will allow the manufacturer to more closely approach 100% efficiency, by increasing the rate and extent of ethanol formation, and in simplifying the biomass ethanol separation by minimizing biomass formation.

The present invention proposes adding an uncoupler of oxidative phosphorylation to a system designed for the production of ethanol, for example, by microorganisms. Such addition will increase both the rate and/or the extent of ethanol formation per unit or biomass from a starting material, with attendant economic benefits for the manufacturer.

As earlier indicated, uncouplers of oxidative phosphorylation are compounds which uncouple the release of chemical energy from the trapping of that chemical energy by cells bioconverting a substrate. Such trapping of energy takes place through the formation of the energy-rich compound adenosine triphosphate, or ATP, and the energy release from a substrate is a result of enzymatic degradation of substrate. Cells normally degrade substrates, trap the released energy in ATP, and use the energy in the ATP to drive biosynthetic or other energy-requiring reactions. When this process is uncoupled, the released energy is instead dissipated as heat. The microorganisms respond by increasing their rate of bioconversion in an attempt to. increase their rate of formation of ATP. In an optimum uncoupled state, the substrate is degraded while no new biomass is formed. As a result, the substrate is more efficiently converted to endproducts rather than new cells.

Examples of uncouplers of oxidative phosphorylation for both waste water treatment and for use in the production of commercial chemicals include bis (tricholoromethyl) sulfone; 4, 7 diphenyl-1, 10 phenathroline; 2-mercaptobenzothiazole; myristic acid; N,N,N',N' tetramethyl p-phenylene diamine; rotenone; salicylanilide; tetraphenylboron; thiosalicylic acid; dicyclohexyl-carbodiimide; B-bromo-B-nitrostyrene; and mixtures thereof. The above is merely exemplary of the type compounds that may be used. Any compound or mixtures thereof capable of uncoupling oxidative phosphorylation will be effective in enhancing product formation as well as waste assimilation in any biodegradative system. As earlier indicated, mixtures of uncouplers may be used, such as a composition comprising at least two members selected from the group bis(tricholoromethyl) sulfone; 4, 7 diphenyl-1, 10 phenathroline; 2-mercaptobenzothiazole; myristic acid; N,N,N',N' tetramethyl p-phenylene diamine; rotenone; salicylanilide; tetraphenylboron; thiosalicylic acid; dicyclohexyl-carbodiimide; B-bromo-B-nitrostyrene; etc.

As earlier indicated, the oxidative uncouplers represent somewhat of an anomaly since they operate quite differently depending upon the dosage. At higher dosages each can act as a toxicant for a microbial population while when used at lower dosages, the objectives of the present invention are achieved. Accordingly, critical concern must be given to assure the use of non-toxicant dosages, since many uncouplers are also industrial biocides.

Therefore, the amount of chemical (uncoupler) used is such as to not totally block the transfer of the energy produced to the energy reservoir of the cells, thus permitting the cells to continue to live and bioconvert the organic substrate. Obviously the foregoing is equally applicable to systems where bioconversion is used to produce commercial products as well as to waste treatment systems where pollutant removal is the objective.

The main resulting effects of uncoupler exposure have been shown to be decreases in biomass yields and increased organic carbon removal. When translated into the terms of a biological wastewater treatment operation, this represents decreased waste sludge production and increased pollutant removal over what could be accomplished under normal controlled conditions. Again, because uncoupler action is aimed at biochemical targets, there is a limit in the useful concentration range due to eventual toxicity. This effective range varies among uncouplers. The most efficient uncouplers can inhibit in the nanomolar range, whereas the less efficient ones are effective only in the millimolar range.

Model waste treatment systems showed that in simplified activated sludge units using one organism and a single carbon source, a steady state operation could be maintained during uncoupler exposure with concomitant biomass yield reductions.

Batch analysis of biomass yield showed uncouplers caused significant (up to 40%) yield reductions over the controls with little or no inhibition of carbon removal efficiencies.

Fermentation system analysis showed that uncouplers can be effective, not only in biodegradation systems, but in biosynthesis operations as well. Increases in carbon breakdown rate, product formation rate, product yields/unit of biomass and product yield/-unit of substrate metabolized were observed with a simultaneous decrease in biomass formation.

EXAMPLE

The present invention will be further described with reference to the accompanying example.

FIRST SERIES OF EXPERIMENTS

The first series of experiments involved the measurement of the amount of $CO_2$ evolved from aerobically growing cultures of the representative organism Klebsiella in the presence and absence of uncoupling agents. The known uncouplers 2, 4 dinitrophenol (DNP) and carbonyl cyanide m-chlorophenyl hydrazone (CCCP) were tested along with bis (tricholoromethyl) sulfone (TCMS) and beta-bromo-beta-nitrostyrene (BNS). It can be seen from the data that CCCP caused large increases in $CO_2$ evolution over the untreated controls at all concentrations used, with the largest increase (29%) at 5 $\mu$g/mL (24.4 uM). There was also no toxic effect seen in the series, as the bacterial counts at 50 $\mu$g/mL were the same as the control levels. TCMS exhibited no uncoupling property, causing only control level toxic effects for all concentrations. Bacterial counts also reflected the lack of uncoupling activity. The uncoupler DNP did not cause significant increases in $CO_2$ blowoff until at least in concentrations greater than 5 ppm (27 $\mu$M). Bacterial counts also reflect that no toxic effects were seen even at 50 ppm. Largest

-13-

increase in $CO_2$ blowoff came at 50 ppm (32%). BNS gave a mixed pattern as opposed to those of DNP and CCCP. Increased $CO_2$ blowoff was seen with concentrations of 0.5 µg/mL to 10 µg/mL (2.2 - 44 µM) with the largest increase at 1.0 µg/mL (13%). However, whereas DNP and CCCP required much higher concentrations, and at least were not toxic at higher concentrations, BNS proved to be quite toxic. Even at 10 µg/mL, the bacterial count was reduced ∿ 50% while still giving greater $CO_2$ blowoff than control cultures. This indicated that while BNS did give the desired result, it also gave only an initially limited range of concentrations within which we could work.

These patterns were the results of Klebsiella cultures having been grown on glucose ($^{14}$C-label). In nature, and in waste treatment facilities, many organic carbon compounds, both simple and complex, would be available for degradation. The known uncouplers and BNS were tested for their ability to increase $CO_2$ evolution from cultures exposed to the common substrates lactate, glycine, glycerol, and a denatured protein mixture. From the data it is apparent that amino acids and protein substrates do not result in as much production of $CO_2$ by Klebsiella and are more likely not utilized as energy sources by the organism or not utilized at all. This was expected in the case of glycine but it was thought that a more complex protein might be degraded and portions used for some energyproduction. It was hoped that these compounds might be a more representative group (more abundant in waste treatment) than simple sugars for determining the extent of activity of BNS. BNS and the known uncouplers showed either no effect or a toxic effect when glycine was used as the labeled substrate. Lactate, a common intermediate in microbial metabolism, and a compound which would normally be used as an oxidizable substrate for running the cytochrome chain, gave a more representative response. BNS treated cultures gave increased $CO_2$ blowoff (an increase as high as 130% at 5 μg/mL) as did both known uncouplers. BNS appears to be more effective than DNP (83% increase) but less effective than CCCP (151% increase). BNS gave increased $CO_2$ blowoff even up to 10 μg/mL concentrations; but the pattern seemingly indicated that the BNS would be toxic at concentrations much greater than that. When glycerol was used as the labeled substrate, BNS treated cultures gave increased $CO_2$ at concentrations up to 5 μg/mL. 10 μg/mL was toxic and seriously curtailed $CO_2$ evolution. Again, BNS was about equal to DNP in effect, but somewhat less effective than CCCP.

## Procedure

The procedure utilized to perform the first series of experiments was as follows:

Sealed vials containing 8 mL of minimal salts medium (Composition: $K_2HPO_4$-3g, $KH_2PO_4$-1g, $MgSO_4$-0.2g, $(NH_4)_2SO_4$-1g, D-glucose-5g (optional) in 1 liter. Autoclave and adjust pH to 6.8) lacking any carbon source were each injected with 1 mL of a suspension of _Klebsiella pneumoniae_. (_Klebsiella_ was grown overnight, shaken at 37°C, in minimal salts medium containing glucose to a heavy cell suspension. The cells were centrifuged at 10,000 g and resuspended to a turbidity of 150 Klett units [Klett-Summerson photometer, #42 blue filter] in minimal salts medium [MSM], no glucose. These cells were again centrifuged, and resuspended in MSM at 1/10 the volume used previously. This 10X cell suspension was used as the standard inoculum for the vials.) The vials were simultaneously injected with 1 mL of a 1 μCi/mL solution of

$^{14}$C-U-D glucose and the appropriate concentration of material to be tested. The compounds tested were fed at concentrations of 0.1, 0.5, 1.0, 5, 10, 25, and 50 $\mu$g/mL. Controls received no test material and all vials were shaken during the course of the experiments at 37°C. Vials were read for $CO_2$ blowoff at the times indicated on the appropriate Tables. Standard bacterial counts were done on each vial after the end of each experimental series. Data were collected on BNS, DNP, TMCS, and CCCP and recorded in the following Tables.

## TABLE 1

### Effect of BNS on the evolution of $CO_2$ by Klebsiella

| | Cumulative Growth Index Reading ($CO_2$ Blowoff) | | | | | | |
|---|---|---|---|---|---|---|---|
| Conc. Of BNS $\mu$g/mL | 0 | 0.5 HR | 1 HR | 2 HR | 4 HR | 5 HR | 5 HR Bacterial Count |
| 0 Control | 6 | 771 | 1,371 | 1,801 | 2,081 | 2,231 | $4.6 \times 10^8$ |
| 0 Control | 5 | 748 | 1,343 | 1,768 | 2,005 | 2,198 | $6.0 \times 10^8$ |
| 0.1 | 20 | 720 | 1,280 | 1,690 | 1,960 | 2,110 | $6.2 \times 10^8$ |
| 0.5 | 33 | 733 | 1,383 | 1,853 | 2,163 | 2,338 | $4.6 \times 10^8$ |
| 1.0 | 24 | 714 | 1,404 | 1,934 | 2,294 | 2,504 | $4.8 \times 10^8$ |
| 5.0 | 6 | 686 | 1,366 | 1,896 | 2,246 | 2,446 | $4.1 \times 10^8$ |
| 10 | 19 | 699 | 1,289 | 1,749 | 2,139 | 2,409 | $2.2 \times 10^8$ |
| 25 | 19 | 400 | 919 | 1,204 | 1,399 | 1,469 | $10^4$ |
| 50 | 12 | 362 | 640 | 834 | 962 | 1,047 | $<10^3$ |
| Sterile Control | 2 | 2 | 6 | 8 | 10 | 13 | -- |

## TABLE 2

Effect of 2, 4, Dinitrophenol (DNP) on the evolution
of $CO_2$ by <u>Klebsiella</u>

<u>Cumulative Growth Index Reading</u>
($CO_2$ Blowoff)

| 2, 4 DNP Conc. μg/mL | 0 HR | 0.5 | 1.0 | 3 | 5 | 5 HR Bacterial Count |
|---|---|---|---|---|---|---|
| 0 Control | 2 | 602 | 922 | 1,412 | 1,712 | $1.9 \times 10^8$ |
| 0 Control | 10 | 600 | 910 | 1,400 | 1,700 | $2.1 \times 10^8$ |
| 0.1 | 4 | 564 | 854 | 1,314 | 1,604 | $2.4 \times 10^8$ |
| 0.5 | 8 | 578 | 888 | 1,398 | 1,718 | $2.0 \times 10^8$ |
| 1.0 | 13 | 613 | 933 | 1,433 | 1,753 | $2.1 \times 10^8$ |
| 5.0 | 3 | 583 | 913 | 1,443 | 1,773 | $2.2 \times 10^8$ |
| 10 | 5 | 705 | 1,065 | 1,665 | 2,025 | $2.6 \times 10^8$ |
| 25 | 6 | 676 | 1,046 | 1,686 | 2,066 | $2.5 \times 10^8$ |
| 50 | 3 | 703 | 1,093 | 1,783 | 2,253 | $3.8 \times 10^8$ |

## TABLE 3

Effect of.TCMS on the evolution of $CO_2$ by Klebsiella

### Cumulative Growth Index Reading
### ($CO_2$ Blowoff)

| TCMS Conc. µg/mL | 0 HR | 0.5 HR | 1 HR | 3 HR | 5 HR | 5 HR Bacterial Count |
|---|---|---|---|---|---|---|
| 0 | 2 | 602 | 922 | 1,412 | 1,712 | $1.9 \times 10^8$ |
| 0 | 10 | 600 | 910 | 1,400 | 1,700 | $2.1 \times 10^8$ |
| 0.1 | 6 | 606 | 946 | 1,486 | 1,806 | $2.8 \times 10^8$ |
| 0.5 | 8 | 548 | 828 | 1,318 | 1,618 | $2.8 \times 10^8$ |
| 1.0 | 7 | 577 | 877 | 1,387 | 1,717 | $2.2 \times 10^8$ |
| 5.0 | 5 | 525 | 815 | 1,305 | 1,615 | $2.0 \times 10^8$ |
| 10 | 6 | 346 | 496 | 886 | 1,196 | $1.3 \times 10^8$ |
| 25 | 6 | 286 | 416 | 626 | 746 | $< 10^3$ |
| 50 | 4 | 284 | 414 | 624 | 749 | $< 10^3$ |

## TABLE 4

Effect of CCCP on the evolution of $CO_2$ by Klebsiella

### Cumulative Growth Index Reading
(CO₂ Blowoff)

| CCCP Conc. $\mu g/mL$ | 0 HR | 0.5 HR | 1 HR | 3 HR | 5 HR | 5 HR Bacterial Count |
|---|---|---|---|---|---|---|
| 0 Control | 5 | 715 | 1,205 | 1,555 | 1,785 | $1.1 \times 10^8$ |
| 0 Control | 6 | 756 | 1,236 | 1,606 | 1,831 | $1 \times 10^8$ |
| 0.1 | 3 | 723 | 1,183 | 1,533 | 1,743 | $7.1 \times 10^7$ |
| 0.5 | 7 | 907 | 1,477 | 1,877 | 2,117 | $1.6 \times 10^8$ |
| 1.0 | 5 | 905 | 1,485 | 1,915 | 2,165 | $1.5 \times 10^8$ |
| 5.0 | 10 | 960 | 1,580 | 2,050 | 2,330 | $2.0 \times 10^8$ |
| 10 | 8 | 908 | 1,448 | 1,808 | 2,008 | $1.5 \times 10^8$ |
| 25 | 11 | 911 | 1,431 | 1,771 | 1,961 | $2.0 \times 10^8$ |
| 50 | 9 | 809 | 1,329 | 1,648 | 1,838 | $2.0 \times 10^8$ |

The Effects of BNS, DNP, and CCCP on the Evolution of
$CO_2$ by Klebsiella grown on Four Different Carbon Sources

The procedure used for determining the $CO_2$ evolution was similar to the procedure described previously. Cells having been grown overnight in MSM and glucose were centrifuged and resuspended in MSM + glucose (cold) and used as inoculum. No other cold carbon source (unlabeled) was added. $^{14}$C-labeled substrates (lactate, glycerol, etc.) obtained from New England Nuclear were used and supplied the radiolabeled carbon for the detection of [14]$CO_2$.

BNS was utilized in concentrations of 0.5 - 10 μg/mL. Fixed concentrations of 5 μg/mL CCCP and DNP were included for comparative purposes. The following data were obtained in these experiments.

## TABLE 5

Effect of uncouplers on the evolution of $CO_2$
by Klebsiella utilizing either Glycerol,
Glycine, Lactate or Denatured Protein

Cumulative Growth Index
($CO_2$ Blowoff)

| Substrate | Conc. Compound | (μg/mL) | 0 HR. | 1 HR. | 3 HR. | 5 HR. |
|---|---|---|---|---|---|---|
| Lactate | BNS | 0 | 2 | 423 | 813 | 1,038 |
| | | 0.5 | 3 | 653 | 1,243 | 1,623 |
| | | 1 | 3 | 643 | 1,243 | 1,623 |
| | | 2 | 3 | 723 | 1,353 | 1,753 |
| | | 5 | 3 | 1,003 | 1,878 | 2,398 |
| | | 10 | 3 | 733 | 1,243 | 1,563 |
| | DNP | 5 | 2 | 722 | 1,473 | 1,903 |
| | CCCP | 5 | 3 | 1,103 | 2,053 | 2,613 |
| Glycine | BNS | 0 | 2 | 502 | 942 | 1,232 |
| | | 0.5 | 1 | 441 | 851 | 1,126 |

-20-

TABLE 5
(Cont'd)

Cumulative Growth Index
(CO₂ Blowoff)

| Substrate | Conc.<br>Compound | (µg/mL) | 0 HR. | 1 HR. | 3 HR. | 5 HR. |
|---|---|---|---|---|---|---|
| | | 1 | 2 | 412 | 782 | 1,007 |
| | | 2 | 1 | 311 | 631 | 841 |
| | | 5 | 1 | 176 | 376 | 506 |
| | | 10 | 1 | 73 | 163 | 219 |
| | DNP | 5 | 1 | 441 | 891 | 1,211 |
| | CCCP | 5 | 1 | 18 | 64 | 114 |
| Glycerol | BNS | 0 | 0 | 561 | 1,151 | 1,521 |
| | | 0.5 | 1 | 670 | 1,420 | 1,910 |
| | | 1 | 2 | 552 | 1,232 | 1,662 |
| | | 2 | 2 | 602 | 1,392 | 1,942 |
| | | 5 | 2 | 422 | 1,192 | 1,662 |
| | | 10 | 1 | 31 | 266 | 436 |
| | DNP | 5 | 0 | 470 | 1,430 | 1,890 |
| | CCCP | 5 | 0 | 670 | 1,420 | 2,020 |
| Denatured | BNS | 0 | 2 | 10 | 22 | 27 |
| Protein | | 0.1 | 2 | 10 | 28 | 33 |
| | | 0.5 | 2 | 10 | 27 | 33 |

## TABLE 5
### (Cont'd)

Cumulative Growth Index
$(CO_2$ Blowoff)

| Substrate | Conc. Compound | $(\mu g/mL)$ | 0 HR. | 1 HR. | 3 HR. | 5 HR. |
|-----------|----------------|-------------|-------|-------|-------|-------|
|           |                | 1           | 2     | 11    | 29    | 36    |
|           |                | 2           | 2     | 9     | 28    | 35    |
|           |                | 5           | 2     | 9     | 26    | 35    |
|           |                | 10          | 2     | 9     | 19    | 25    |

Second Series of Experiments

After the first series of experiments, it was apparent that BNS could be utilized to increase the blowoff of $CO_2$ (breakdown of substrates) while not increasing bacterial mass significantly when used at concentrations of 0.5 - 5 $\mu$g/mL. The desired effect was slight/negligible at concentrations of $<$ 0.5 $\mu$g/mL, and the toxic properties of the compound become evident at concentrations $>$ 5.0 $\mu$g/mL.

Experiments to look at the above conclusions in depth were performed. In these experiments, cultures of Klebsiella were challenged with various concentrations of BNS and the effects on $CO_2$ evolution from glucose, viability, TOC (Total Organic Carbon), TIC (Total Inorganic Carbon) production, removal, and DO (Dissolved Oxygen) removal were determined. Concentrations of BNS from 0.05 to 100 $\mu$g/mL were used. As can be seen from the data, the most rapid (and greatest) amount of $CO_2$ evolution was achieved at a concentration of 0.05 - 0.1 ppm BNS. $CO_2$ in amounts greater than the untreated control were seen at BNS concentrations up to $\sim$2 - 5 $\mu$g/mL. $CO_2$ evolution decreased after 5 $\mu$g/mL. Viability data showed that the cells retained viability equal to the control level at BNS concentrations up to 2 $\mu$g/mL. The viability dropped off after that with 99% inhibition at concentrations somewhere between 10 - 20 $\mu$g/mL. Organic carbon removal gave the most puzzling pattern. Only a concentration of 0.05 ppm showed excessive (more than control) organics removal. BNS concentrations $\geq$ 2 $\mu$g/mL resulted in increases in TOC, probably due to organic carbon added by the death and lysis of the bacterial cells (reflected in the viability data). Organic carbon removal by cultures treated with 0.1 - 1 $\mu$g/mL of BNS showed little or no organic carbon removal and little loss of viability.  $CO_2$ was produced, however, as reflected by the $CO_2$ blowoff experiments and the TIC data.  The organic carbon being oxidized (removed) must have been cancelled out by an organic carbon addition caused by limited bacterial lysis.  This phenomenon was investigated again.  Dissolved oxygen readings showed that oxygen was being consumed at levels approaching control values at BNS concentrations up to 2 ppm and DO removal declined after that.  It appeared that 5 ppm BNS would be the maximum concentration that could be used for the desired effect, with the majority of favorable results occurring in the 0.05 - 2 ppm range.

These data were further looked into by repeating the TOC data in nutrient broth and by repeating the entire range of experiments in an inorganic salts medium containing only glucose as a carbon source.  The latter experiments were run to try to determine the effects of BNS on cultures exposed to a less than optimal type medium (one that more closely resembles nutritionally deficient natural waste waters).

A review of the data on TOC removal by cells grown in nutrient broth shows that the results from both experiments are very similar in most respects.  At 0.05 $\mu$g/mL level, TOC removal was equivalent to the untreated control.  When BNS concentrations of $\geq$ 0.5 $\mu$g/mL were used, TOC removal ceased and in the second series of experiments, TOC levels increased sharply at 0.5 - 2 $\mu$g/mL concentrations of BNS.  Similar results were obtained when BNS was used to treat bacteria growing in the glucose salts medium.  No increases in TOC removal were seen.  The TOC concentration increased rapidly at BNS concentration of 0.5 - 2 ppm only to fall back to control levels at 5 ppm.  The cells then added TOC to the medium again from 5 - 1000 $\mu$g/mL concentrations of BNS.  This middle range spike in

-24-

TOC (0.5 - 2 µg/mL BNS) was thought to be caused by excessive extra cellular polymer sloughing and limited cellular death as evidenced by the viability counts in the 0.5 - 2 µg/mL BNS range. After 5 µg/mL BNS, the cellular death rate was greatly increased and lysis of the cells and the resultant loss of cytoplasm to the medium would explain the massive increases in TOC (along with the now significant contribution made by the BNS itself.) The same pattern of viability retention was seen with the glucose salts medium, although the bacteria were considerably more sensitive in that medium. Whereas significant toxicity of the BNS did not show up until the BNS reached concentrations of ~5 - 10 µg/mL in nutrient broth, the toxic concentration in glucose/salts medium was around 0.5 - 1 µg/mL. No increase in biomass over control levels was noted at any concentration of BNS. The dissolved oxygen concentration was decreased in the presence of BNS at concentrations of 0.05 - 1 µg/mL but at no concentration of BNS was the removal greater than the control level.

This set of experiments tends to make the BNS range of useful concentrations even lower than noted before. Toxic effects were seen at lower concentrations of BNS than in the nutrient broth experiments (0.5 - 1 µg/mL) which greatly restricts the viable concentrations of BNS. DO removal was seriously affected by concentrations of BNS that were $\geq$ 0.5 µg/mL. In short, it would appear that BNS would have to be used at concentrations $\leq$ 0.5 µg/mL in order to prevent the upset of a waste treatment facility by killing off the microbial populations. Based on the above experimentation, the range of use of BNS needed to achieve the desired results without causing toxic effects would have to be 0.05 - 0.5 µg/mL.

## Procedure

The experiments coordinating the measurements of $CO_2$ evolution, TOC reduction, viable count, and dissolved oxygen (DO) reduction were performed as follows:   $CO_2$ evolution by <u>Klebsiella</u> was measured as described previously using a narrowed set of BNS concentrations.  At the same time, <u>Klebsiella</u> cells grown overnight in nutrient broth were standardized into an inoculum of 250 Klett units.  These cells were used to inoculate 19 mL quantities of fresh nutrient broth (1 mL inoculum) in baffled shake flasks.  These shake flasks were placed into water bath shakers at 37°C and allowed to shake for 5 hours.  Concentrations of BNS equivalent to the $CO_2$ blowoff experiment were added to the flasks at the beginning of the experiment.  Controls containing no BNS were included for comparisons.  Total organic carbon measurements were taken after 5 hours incubation.  Viable counts were done at 1 hour, 3 hours, and 5 hours.  A parallel experiment was performed using the same setup except that after BNS additions, the flasks were shaken for 30 minutes and then held stationary.  DO measurements were taken at 5 hours incubation with the use of a YSI Model 54A oxygen meter.  Continued shaking of the flasks would have resulted in saturated $(O_2)$ conditions in the medium, with no detection of $O_2$ depletion by the cultures.

The data developed for each of the studies is recorded in the following Tables:

0162545

-26-

## TABLE 6

Effect of BNS on the evolution of $CO_2$
by <u>Klebsiella</u> utilizing $^{14}C$-D-Glucose

Cumulative Growth Index Reading
($CO_2$ Blowoff)

| BNS Conc. ($\mu g/mL$) | 0 HR | 0.5 HR | 1 HR | 3 HR | 5 HR |
|---|---|---|---|---|---|
| 0 | 2 | 752 | 1,042 | 1,522 | 1,832 |
| 0.05 | 2 | 762 | 1,082 | 1,642 | 2,012 |
| 0.1 | 2 | 877 | 1,197 | 1,797 | 2,197 |
| 0.5 | 1 | 801 | 1,121 | 1,761 | 2,201 |
| 1.0 | 1 | 761 | 1,091 | 1,741 | 2,201 |
| 2 | 2 | 752 | 1,072 | 1,732 | 2,212 |
| 5 | 0 | 750 | 1,032 | 1,570 | 1,980 |
| 10 | 2 | 682 | 952 | 1,392 | 1,662 |
| 20 | 1 | 451 | 616 | 916 | 1,101 |
| 50 | 1 | 271 | 381 | 651 | 876 |
| 100 | 2 | 97 | 202 | 287 | 365 |

## TABLE 7

Total Organic Carbon (TOC) Removal
by Klebsiella under BNS Influence (5 Hours)

| BNS Conc. (μg/mL) | Total Carbon (μg/mL) | Tot. Inorg. Carbon (μg/mL) | Tot. Org. Carbon (μg/mL) | % Org. Carbon Removal |
|---|---|---|---|---|
| 0 | 2,600 | 145 | 2,455 | 30% |
| 0.05 | 2,350 | 145 | 2,205 | 37% |
| 0.1 | 4,000 | 155 | 3,845 | +9% |
| 0.5 | 3,600 | 145 | 3,455 | 1% |
| 1.0 | 3,400 | 120 | 3,280 | 6% |
| 2 | 4,150 | 96 | 4,054 | +16% |
| 5 | 4,800 | 63 | 4,737 | +35% |
| 10 | 3,900 | 12 | 3,888 | +11% |
| 20 | 4,200 | 9 | 4,191 | +19% |
| 50 | 4,600 | 7 | 4,593 | +31% |
| 100 | 7,100 | 7 | 7,093 | +102% |
| 0, Sterile Control | 3,500 | Negligible | 3,500 | - |

## TABLE 8

### Viable Count of Klebsiella Cultures treated with BNS

Log$_{10}$ Cell Concentration

| BNS Conc. (µg/mL) | 0 HR | 1 HR | 3 HR | 5 HR |
|---|---|---|---|---|
| 0 | 8.04 | 8.20 | 8.59 | 9.06 |
| 0.05 | | 8.17 | 8.68 | 9.10 |
| 0.1 | | 7.97 | 8.67 | 9.03 |
| 0.5 | | 8.18 | 8.54 | 9.11 |
| 1.0 | | 8.20 | 8.52 | 8.97 |
| 2 | | 8.03 | 8.46 | 9.03 |
| 5 | | 8.06 | 8.23 | 8.67 |
| 10 | | 7.42 | 7.29 | 7.35 |
| 20 | | 6.29 | 6.05 | 4.89 |
| 50 | | 6.17 | 3.30 | <3.0 |
| 100 | | 4.88 | <3.0 | <3.0 |

## TABLE 9

Dissolved Oxygen (DO) Removed by Klebsiella
Treated with BNS (5 HR. Contact)

| BNS Conc. (µg/mL) | DO Conc. (µg/mL) @ 5 Hrs. |
|---|---|
| Starting Nutrient Broth | 6.8 |
| Sterile Control | 6.8 |
| Klebsiella Control | 5.1 |
| 0.05 | 6.0 |
| 0.1 | 6.0 |
| 0.5 | 5.7 |
| 1.0 | 5.7 |
| 2 | 5.8 |
| 5 | 6.4 |
| 10 | 7.0 |
| 20 | 6.8 |
| 50 | 6.8 |
| 100 | 6.8 |

-30-

An experiment similar in design to the previous one was performed utilizing salts medium (glucose as sole carbon source) as the growth medium (replacing nutrient broth). Dissolved oxygen (DO), viable counts, and TOC measurements were taken at the indicated times and appear in Tables 10 - 12.

## TABLE 10

Dissolved Oxygen Removed By <u>Klebsiella</u> treated
with BNS (5 Hours Contact)

| BNS Conc. ($\mu$g/mL) | DO Conc. ($\mu$g/mL) @ 5 hrs. | % Removal |
|---|---|---|
| Initial Salts Medium (Sterile) | 6.8 | - |
| 5 hour Control | 4.2 | 38 |
| 0.05 | 4.3 | 37 |
| 0.1 | 4.8 | 29 |
| 0.5 | 5.8 | 15 |
| 1 | 6.2 | 9 |
| 2 | 6.7 | 0 |
| 5 | 6.7 | 0 |
| 10 | 6.7 | 0 |
| 20 | 6.8 | 0 |
| 50 | 6.8 | 0 |
| 100 | 6.8 | 0 |

## TABLE 11

### Viable Count of <u>Klebsiella</u> cultures treated with BNS

Log$_{10}$ Cell Concentration

| BNS Conc. (μg/mL) | 0 HR. | 3 HR. | 5 HR. |
|---|---|---|---|
| 0 | 7.80 | 8.25 | 8.80 |
| 0.05 | | 8.25 | 8.87 |
| 0.1 | | 8.25 | 8.74 |
| 0.5 | | 8.04 | 8.23 |
| 1 | | 7.72 | 7.81 |
| 2 | | 6.58 | 6.21 |
| 5 | | <3 | <3 |
| 10 | | <3 | <3 |
| 20 | | <3 | <3 |
| 50 | | <3 | <3 |
| 100 | | <3 | <3 |

## TABLE 12

Total Organic Carbon Removal By Klebsiella under
BNS influence (5 Hours) in Two Different Media.

### Nutrient Broth

| BNS Conc. (μg/mL) | Total Carbon | Tot. Inorg. Carbon | T.O.C. | % Organic Carbon Removed |
|---|---|---|---|---|
| Sterile Control | 3,200 | 2 | 3,198 | - |
| Control (untreated) | 2,550 | 145 | 2,405 | 25% |
| 0.05 | 2,700 | 140 | 2,560 | 20% |
| 0.1 | 2,750 | 140 | 2,610 | 18% |
| 0.5 | 3,600 | 145 | 3,455 | 0 |
| 1 | 4,200 | 135 | 4,065 | 0 |
| 2 | 6,200 | 125 | 6,075 | 0 |
| 5 | 3,350 | 74 | 3,276 | 0 |
| 10 | 3,600 | 6 | 3,594 | 0 |
| 20 | 3,750 | 5 | 3,745 | 0 |
| 50 | 4,700 | 4 | 4,696 | 0 |
| 100 | 7,400 | 3 | 7,397 | 0 |

TABLE 12
(Cont'd)

Glucose/Salts Medium

| BNS Conc. (µg/mL) | Total Carbon | Tot. Inorg. Carbon | T.O.C. | % Organic Carbon Removed |
|---|---|---|---|---|
| Sterile Control | 2,500 | 8 | 2,492 | - |
| Control (untreated) | 1,700 | 8 | 1,692 | 32% |
| 0.05 | 1,800 | 7 | 1,793 | 28% |
| 0.1 | 1,900 | 5 | 1,895 | 24% |
| 0.5 | 3,100 | 9 | 3,091 | 0 |
| 1.0 | 4,400 | 9 | 4,391 | 0 |
| 2 | 7,200 | 8 | 7,192 | 0 |
| 5 | 2,175 | 8 | 2,167 | 13% |
| 10 | 2,750 | 9 | 2,741 | 0 |
| 20 | 2,900 | 8 | 2,892 | 0 |
| 50 | 4,300 | 6 | 4,294 | 0 |
| 100 | 7,000 | 8 | 6,992 | 0 |

The foregoing experiments were used as a guideline for determining the necessary concentrations which should be used in a continuous flow through system (model activated sludge system). Even though the BNS concentrations necessary for detecting useful activity (and not toxic) were defined, it was not possible to absolutely predict what concentrations would be necessary for a complicated continuous flow system. Two separate attempts were made to conduct continuous flow studies, but each attempt failed because of poor model design and the inability with the equipment available to monitor and maintain control of the system, treatments, microbial population etc.

## Additional Studies

A variety of direct and indirect measurements were employed to assess the potential of chemical additives to enhance the performance of biologically sponsored wastewater treatment. Enhancement was defined as causing lower sludge yields for disposal while improving organic removal efficiency.

Because of the inherent toxicity of the molecules chosen for study, close attention was payed to possible impairment of waste treatment performance, as well as enhancement. Therefore, all assays of test system performance in this study can be used to judge inhibition as well as enhancement.

## Screening of Known Uncouplers

Many oxidative uncouplers have been identified in the literature, and from prior work done by the inventors. Their

attempts to investigate uncouplers as a    a class used $^{14}CO_2$ evolution measurements to determine whether    uncouplers show similar ranges between the effective and the t    toxic doses, and whether the performance of the uncoupler was affec    ted by the substrate being metabolized.

Screening of the known uncou    uplers has shown measurement of $^{14}CO_2$ production to be a valuable scre    ening tool to assess the toxicity range and relative activity a    among uncouplers.

It is evident from the scree    ning data recorded in the Tables which follow that the toxicity    of uncouplers varies considerably. Differences in relative unc    coupling activity among compounds is dependent upon the type of s    substrate being consumed. Some compounds (i.e., CCCP, dicyclohexylcar    rbodiimide) were toxic at 10 umol. on some substrates, where others    (tributylamine, tetraphenyl boron) were not toxic at 100 umol. Bis    s-trichloromethyl sulfone (TCMS) exhibited good activity at 10 u    umol concentration on all four substrates studied but was not further    investigated since it does not readily hydrolyze. Ranking of unc    coupler activity on radiolabeled glucose was as follows: BNS >    salicylanilide > thiosalicylic acid > rotenone. When radio    labeled glycerol was employed as a sole carbon source, the following    activity ranking resulted: salicylanilide > BNS > rotenone > thio    salicylic acid. The tetraphenylboron (Na) had essentially no re    sponse on glucose and mixed activity on glycerol. Salicylanilide    on both substrates had the broadest range of activity with no    toxicity.

Variability of uncoupler-med    iated response with substrate could be linked to differences in ener    rgy yield from these carbon sources, chemical interactions between    uncoupler and the culture

-36-

media, differences in growth rate on the different substrates, interactions associated with cellular substrate transport, membrane binding of the uncouplers or differing energy expenditures between active transport (for glucose) and passive diffusion (for glycerol).

Concern over uncoupler activity dependence upon substrate character are warranted in that wastewater characteristics are never universal, nor even consistent within one treatment system. Composition can vary from that of a single carbon source in specialized industry to complex mixtures of organics seen in municipalities or in diversified manufacturing or production operations. Thus, the showings that the substrate is an important variable suggested that further work would be necessary to understand uncoupler performance in mixed, rather than single, substrate system.

## TABLE 13

### % of $^{14}CO_2$ Product From Control (K. pneumoniae IPC 500)

| COMPOUND | D-Glucose | | D-Galactose | | Glycerol | | Pyruvate | |
|---|---|---|---|---|---|---|---|---|
| | 10 μm | 100 μm | 10 μm | 100 μm | 10 μm | 100 μm | 10 μm | 100 μm |
| – Myristic Acid | + 23.6% | + 0.7% | – 1.8% | + 2.7% | – 16.5% | – 15.8% | – 1.5% | – 14.4% |
| – N, N'Dicyclohexyl carbodiimide | + 17.6% | – 56.6% | – 99.5% | – 99.2% | – 95.2% | – 96.0% | + 11.4% | – 122.3% |
| – Bis-Trichloromethyl Sulfone | + 29.9% | – 86.1% | + 2.3% | – 98.1% | + 21.6% | – 99.6% | + 8.2% | – 105.4% |
| – BNS* | + 21.4% | – 94.3% | – 19.5% | – 99.3% | + 18.5% | – 99.6% | + 17.3% | – 87.5% |
| – CCCP | + 5.7% | – 52.5% | – 59.2% | – 99.5% | + 3.3% | – 53.2% | + 15.4% | – 76.0% |
| – Deoxychloric Acid | – 0.5% | + 8.2% | – 3.5% | + 3.8% | – 4.8% | – 0.5% | – 7.0% | – 2.6% |
| – 4, 7 Diphenyl 1, 10 phenan-throline | – 1.3% | + 34.8% | – 99.4% | – 99.6% | – 96.4% | – 97.8% | + 6.0% | – 60.4% |
| – 2 mercaptoben-zothiazole | + 22.8% | + 29.9% | + 1.4% | – 12.8% | + 1.7% | – 8.9% | + 4.0% | + 7.1% |

## TABLE 13
### (Cont'd)

% of $^{14}CO_2$ Product From Control (K. pneumoniae 1PC 500)

| COMPOUND | D-Glucose | | D-Galactose | | Glycerol | | Pyruvate | |
|---|---|---|---|---|---|---|---|---|
| | 10 μm | 100 μm | 10 μm | 100 μm | 10 μm | 100 μm | 10 μm | 100 μm |
| - N,N,N',N'-Tetra-methyl p-phenylenediamine | + 5.2% | + 15.2% | - 2.3% | - 89.2% | + 9.2% | - 20.3% | + 7.4% | - 1.5% |
| - Rotenone* | + 0.7% | + 10.6% | + 9.1% | + 1.1% | - 2.7% | + 3.5% | - 16.6% | + 2.2% |
| - Salicylanilide* | + 1.8% | - 37.2% | - 4.3% | - 94.7% | + 18.3% | - 17.5% | + 4.0% | - 22.7% |
| - Tetraphenylboron (Na)* | - 4.8% | - 1.4% | + 0.1% | - 0.3% | - 2.2% | + 10.5% | - 4.2% | + 6.4% |
| - Tributylamine | - 0.9% | + 5.2% | - 1.3% | + 6.7% | - 9.4% | + 1.3% | - 2.3% | + 3.6% |
| - Thiosalicylic acid* | - 3.2% | + 16.9% | 0.0% | + 4.2% | - 1.4% | + 26.8% | + 1.5% | - 3.0% |

* - Expanded dosage range run

-38-

-39

## TABLE 14

Expanded Dose Response Range on Glucose and Glycerol
(K. pneumoniae 1PC 500)

$\triangle$% of $^{14}CO_2$ Prod. From Control

| COMPOUND | DOSE (µM) | D-GLUCOSE | DOSE (µM) | GLYCEROL |
|---|---|---|---|---|
| Bromonitrostyrene | 1 | + 6.1% | 1 | − 9.3% |
| | 5 | + 9.0% | 5 | + 7.7% |
| | 10 | + 20.1% | 10 | + 4.2% |
| | 15 | − 7.7% | 15 | + 14.8% |
| Rotenone | 50 | + 9.8% | 5 | + 2.8% |
| | 100 | + 7.5% | 10 | + 2.9% |
| | 150 | − 8.9% | 50 | + 5.1% |
| | 200 | + 12.6% | 70 | + 5.3% |
| Salicylanilide | 1 | + 11.8% | 1 | + 4.3% |
| | 5 | + 8.7% | 5 | + 14.8% |
| | 10 | + 12.0% | 10 | + 21.5% |
| | 50 | + 34.8% | 50 | + 17.3% |
| Tetraphenylboron (Na) | 1 | + 0.9% | 10 | + 14.6% |
| | 10 | − 2.6% | 100 | − 11.6% |
| | 100 | − 1.0% | 150 | + 7.8% |
| | 200 | − 2.8% | 200 | − 13.8% |
| Thiosalicylic Acid | 10 | − 2.1% | 10 | − 2.6% |
| | 100 | + 12.1% | 50 | − 1.0% |
| | 150 | + 8.0% | 70 | + 5.9% |
| | 200 | + 15.7% | 100 | + 7.8% |

## Biomass Yield Studies

Experiments were conducted to demonstrate that carbon removal efficiencies would remain high even in the face of reduced biomass yields. These studies were done with several uncouplers, and biomass yield studies in both batch and continuous culture have shown that yields can be reduced without affecting carbon removal efficiencies.

### Effect of Uncouplers on Batch and Continuous Cultures of E. coli K12

Media component composition for batch and continuous culture experimentation was as follows:

#### Vogel Bonner Medium E 25X (VBE)

| | g/l |
|---|---|
| $MgSO_4 \cdot 7H_2O$ | 5.0 |
| Citric acid $\cdot H_2O$ | 50.0 |
| $K_2HPO_4$ (anhydrous) | 250.0 |
| $NaNH_4HPO_4 \cdot 4 H_2O$ | 87.5 |

#### Trace Element Mixture

| | g/l |
|---|---|
| CaCl | 4.40g |
| $ZnSO_4$ | 0.82g |
| $MnCl_2$ 4 $H_2O$ | 2.00g |
| $FeCl_2$ 6 $H_2O$ | 5.40g |
| $CuSO_4$ | 0.16g |
| $CoCl_2$ 6 $H_2O$ | 0.40g |
| $H_3BO_4$ | 0.06g |
| $NaMoO_4$ | 0.04g |
| Conc. HCl | 10.0ml |

## Biomass Yield Determinations

Measurement of biomass yield and carbon uptake rates and removal efficiencies were measured in both batch and continuous culture systems.

Biomass determinations were by Millipore Filtration using Millipore type H.A. 0.45 $\mu$ filters. Samples were centrifuged @ 15,000 x for 10 min., supernatant was filtered through a dried and preweighed filter. The centrifuged pellet was then vortexed in 2 mL of D.I. water and filtered also. Samples were then dried overnight @ 105°C and re-weighed. Biomass was the weight of the dried filter less the tared filter weight.

Glucose determinations were carried out on the centrifuged reactor sample supernatants using glucose enzymatic assay (hexokinase and glucose 6-phosphate dehydrogenase) UV-15 Sigma Chemical Co., St. Louis, MO. (Assay set-up pamphlet accompanies kit). Spectrophotometric measurements were run on a Zeiss PM-6 Spektralphotometer, Carl Zeiss, 7082 Oberkochen, West Germany.

## Batch Analysis

Escherichia coli K12 ATCC 25404 was grown to 175 K.U. (after 48 hours) on 10 g/l dextrose, 75 mL/l 25x VBE, 5 mL/l trace mixture. Ten mL of this culture was then used as seed for 300 mL of media of the same composition and dosed with varying concentrations of CCCP, myristic acid and salicylanilide.

Initial and 72 hour measurements for glucose and biomass were run by the previously described methods.

## Continuous Culture Yields

Continuous culture medium was composed of 5 g/l glucose, 40 mL/l 25 X VBE, and 2.5 mL/l trace mixture. Bulk medium composed of deionized $H_2O$ plus 25X VBE was steam sterilized for 45 min. @ 121°C. Trace mix was autoclaved separately and added aseptically. Glucose was filter sterilized and added last (aseptically).

Bioflo Model C32 Chemostat units (bioreactors), New Brunswick Scientific, Edison NJ, with a working volume of 1400 mL were utilized for the continuous culture experimentation. pH control on the systems was maintained by Model 5997-20 Horizon pH Controllers. Ingold Sterilizable pH Electrodes, Type 465-35-K9, Andover, MA, were sterilized in place before each run. pH adjustment was by controller addition of sterile 2N NaOH upon demand.

Sterile chemostats vessels were filled to working volume with media seeded with 10 mL of 48 hr. E. coli K12 ATCC 25404 culture grown up overnight in nutrient broth (BBL, Cockeysville, MD) @ 37°C. Reactors were run for 72 hours in the batch mode @ 37°C, and then put into continuous flow. The reactors were run for a minimum of five reactor turnovers before initial sampling to insure a steady state. Sampling was then conducted at a maximum of once/- reactor turnover, with a minimum number of 11 reactor turnovers per chemostat run. Both control and BNS containing chemostats were stripped, cleaned and the E. coli culture reestablished (as above) each time before either the BNS concentration or the dilution rate was increased. Eleven reactor turnovers is equivalent to 3.5 days at the highest dilution rate used (minimum time for a chemostat run).

## Uncoupler Addition to Continuous Culture

BNS stock was composed of 10% BNS, 5% Triton X-114 and 85% carbitol acetate. BNS was diluted in deionized water to the appropriate 10X concentration desired. Breakdown kinetics were measured in a deionized $H_2O$/BNS system by spectrophotometric measurement at 320 nm and a BNS half life was determined to be 7 hours. Continuous and separate pumping of uncoupler was by a Masterflex 7552-10 peristalic pump (Cole Parmer, Chicago, IL) through silicon tubing lines into the bioreactor. Feed flow was split 90% organic feed, 10% BNS feed for total flow. New uncoupler feed was made up daily from the previously mentioned stock, since there was only 10% of the original BNS concentration left before the new daily solution of BNS feed was begun. BNS dose of 1, 2 and 5 ppm was based on the total bulk feed. Reservoir solutions were mixed constantly on a Fisher versamix magnetic stir plate at a speed of 0.4/1.0 to prevent settling out of chemical.

Chemostat system parameters were set at pH - 7.0, temperature 37°C, aeration 2.0 standard litres per minute (SLPM) and agitation at 300 rpm for all experimentation.

Daily checks were made on system for pH, media flow, NaOH consumption, BNS flow, temperature, agitation speed and aeration rate. Mixed liquor glucose and gravimetric biomass determinations were performed at least 5 times per week. Periodic checks were also made on the organic strength of the influent medium, depending on the frequency of new media make-up.

Sterility checks were made microscopically and on selective and nonselective solid media. Cultural streaks from each reactor were made approximately every 3 days on Levine EMB agar (BBL, Cockeysville, MD) and on half-strength nutrient agar.

External temperature control was supplied by a Model 5127 temperature controller, (Cole Parmer Instrument Co., Chicago, IL) and a 650W immersion heater when needed.

The data developed for both the batch and continuous series of evaluations are recorded in the following Tables 15 and 16.

### TABLE 15

Batch Analysis of Uncoupler Response
(E. coli K12 ATCC 25404)

| | Yield (g cells/g glucose) | % Yield From Control | Glucose removal Efficiency (72 Hrs.) |
|---|---|---|---|
| Control | 0.082 | -- | 100% |
| CCCP ( 0.2 ppm) | 0.071 | -13.4% | 100% |
| ( 0.4 ppm) | 0.070 | -14.6% | 100% |
| ( 1.0 ppm) | 0.059 | -28.0% | 98.2% (-1.8%) |
| ( 1.9 ppm) | 0.047 | -42.7% | 95.2% (-4.8%) |
| Myristic Acid | | | |
| ( 5.0 ppm) | 0.082 | 0.0% | 100% |
| (10.0 ppm) | 0.072 | -12.2% | 100% |
| (25.0 ppm) | 0.066 | -19.5% | 100% |
| Salicylanilide | | | |
| ( 5.1 ppm) | 0.072 | -12.2% | 100% |
| (10.1 ppm) | 0.077 | - 6.1 | 100% |
| (25.3 ppm) | 0.078 | - 4.9% | 99.9% (-0.1%) |
| (50.5 ppm) | 0.049 | -40.2% | 99.7% (-0.3%) |

## Batch Analysis

Table 15 shows values of yield for E. coli K12 ATCC 25404 when exposed to increasing concentrations of CCCP, myristic acid and salicylanilide. Corresponding % reductions in cell yield and glucose removal efficiencies are also shown. As predicted, biomass yield decreased while substrate removal remained virtually unchanged.

Batch data revealed that 0.4 ppm of CCCP could facilitate a 14.6% drop in biomass yield while still maintaining glucose removal efficiency at 100%. 1.0 ppm of CCCP began to inhibit carbon removal slightly (98.2% efficiency) with a 28% drop in biomass yield. At 1.9 ppm of CCCP, a 42.7% drop in biomass yield and 95.2% glucose removal efficiency was seen (4.8% drop from control). Myristic acid showed no inhibitory effects on glucose removal up to 25 ppm where a 19.5% reduction in biomass yield was realized. Salicylanilide at 50.5 ppm showed up to a 40.2% drop in biomass yield while glucose removal efficiency was maintained at 99.7%. While it is unlikely to be reproduced in a natural field situation, these results suggest that biomass or sludge could be reduced up to 40% without affecting waste system performance. It can also be seen from these data, as well as the general screening data previously mentioned, that bacterial toxicity ranges vary between uncouplers. This has been attributed in part to differences in the lipophilicity or the partitioning coefficients of the molecules tested.

## TABLE 16

Response of E. coli K12 ATCC 25404 to the oxidative
uncoupler BNS in the continuous culture mode

| ppm BNS | Dilution Rate (hr.$^{-1}$) | Biomass Yield (g cells/g substrate) | % From Control |
|---|---|---|---|
| 0.0 | 0.060 | 0.316 | -- |
| 0.0 | 0.086 | 0.325 | -- |
| 0.0 | 0.131 | 0.337 | -- |
| 1.0 | 0.068 | 0.268 | -15.2% |
| 1.0 | 0.086 | 0.276 | -15.0% |
| 1.0 | 0.132 | 0.324 | - 3.9% |
| 2.0 | 0.066 | 0.273 | -13.3% |
| 2.0 | 0.090 | 0.241 | -26.1% |
| 2.0 | 0.130 | 0.275 | -18.3% |
| 5.0 | 0.070 | 0.287 | - 9.5% |
| 5.0 | 0.090 | 0.237 | -27.3% |
| 5.0 | 0.131 | 0.298 | -11.5% |

Yield comparisons can be seen in Table 16 for 1.0 ppm, 2.0
ppm and 5.0 ppm BNS. These data again show that BNS affects the
yield, but they further show that the yield variation is a function
of the dilution rate, and, hence, the growth rate.

## Metabolic By-Product Effects

Metabolic by-product formation in an uncoupled batch
system was analyzed in a yeast-alcohol fermentation.

Saccharomyces cerevisiae (Institute of Marine Sciences,
U. Of Miami Culture Collection) was used in the following fermentation medium: 1% glucose, 13.4 g/l yeast nitrogen Base (BBL, Cockeysville, MD), 0.5 g/l sodium thioglycollate. pH was 5.68, temperature 33°C, agitation 8.5/10 on Fisher Versamix stirplate. CCCP was dosed at 2.0 mg/l.

Reaction vessel was a 3.0 liter working volume fermenter jar - New Brunswick Scientific. Temperature control was held by recirculation of a 33°C hot water bath through the reactor heating coils by a Little Giant Model 1 submergible pump (Little Giant Pump Co., Oklahoma City, OK).

Glucose and biomass determinations were made by the previously mentioned techniques. Ethanol determinations was by the alcohol dehydrogenase method 331-UV (Sigma Chemical Co., St. Louis, MO). Reactors were run for approximately 150 hours with sampling times varying throughout the experiment.

G. C. analysis on the fermentation end products (metabolites) was for volatile and non-volatile fatty acids (VPI Anaerobe Manual, Blacksburg, VA). Analysis was on a SP 1220 1/8" x 6 ft. stainless steel column (Supelco, Bellefonte, PA) using a Beckman GC-M gas chromatograph equipped with a Flame Ionization detector.

Fermentation System Analysis

Ethanol production, glucose removal and biomass production in a yeast fermentation system are compared in Table 17 which follows. These results show that CCCP increased the ethanol yield per cell almost an order of magnitude, combining an increasing yield with a reduced cell crop.

## TABLE 17

### Response of a Yeast-ETOH Fermentation To Uncoupler Dosing

| | $\dfrac{\text{moles ETOH/}}{\text{mole glucose}_f}$* | $\dfrac{\text{mole ETOH/Mole Glucose}_f}{\text{g cells}}$ | $\dfrac{\text{g ETOH formed}}{\text{hr. max.}}$ | $\dfrac{\text{g cells}}{\text{mole glucose}_f}$ | $\dfrac{\text{g glucose}}{\text{consumed/hr. max.}}$ |
|---|---|---|---|---|---|
| Control | 1.27 | 1.96 | 0.32 | 11.78 | 0.21 |
| 2.0 ppm CCCP | 1.39 (+9.4%) | 4.02 (+105.1%) | 0.45 (+41.6%) | 6.59 (-44.1%) | 0.39 (+88%) |

*f = final

## FIELD TESTING

To ascertain the viability of the inventive concept, a field evaluation was conducted utilizing actual waste water. The particulars were as follows:

Experimental bioreactors were constructed using Nalgene 200 liter heavy duty, linear polyethylene cylindrical tanks with fitted cover (VWR Scientific). Incorporated into the design were three evenly spaced 4" x 34" polyethylene baffles, and a fitted P.V.C. aeration ring on the reactor bottom. Aeration and mixing were carried out by employing a Millipore 1/6 HP, 115 V, 4.4 amp, 60 Hz vacuum/pressure pump (Millipore Corp.) delivering 1.2 SCF/-minute. Mixing was also facilitated by a Lightnin Model K-4520-20 variable speed mixer with two, three blade turbine impellers with 1inch blades on a 24" stainless steel shaft (Cole Parmer). Mixers were fitted into the reactor cover.

Substrate was delivered to the parallel reactors by pumping mixed liquor from the number one, out of four, in-series, aeration basin at a Regional Sewerage Authority, located in NJ. The pump was a Model 7553-10 Cole Parmer Masterflex peristaltic pump with multiple Masterflex 7016 series heads and silicon pump tubing. Reactors were run on a 10-day solids and hydraulic retention time in the fed-batch mode.

B-bromo B-nitrostyrene was obtained from Givaudan Corporation and used in a formulation of 10% BNS, 5% Triton X-114, and 85% carbitol acetate. BNS dosing was by shot-feeding on a Monday, Wednesday, Friday schedule as was reactor drawdown, sample collection, and reactor maintenance.

Biomass solids analysis was done by Millipore filtration using type H.A., 0.45 μ filters (Millipore Corporation). Collected samples were centrifuged @ 15,000xg for 10 minutes. Supernatant was filtered through a dried and preweighed filter. The centrifuged pellet was then vortexed briefly in 2 mL of deionized water and filtered in a similar fashion. Samples were then dried @ 105°C overnight and reweighed. Biomass solids were the weight of the dried sample less the tare weight.

Analysis on soluble effluent components was carried out on pre-clarified and "deep frozen" samples. Collected samples were clarified by settling for 30 minutes, centrifuging supernatant @ 10000xg for 10 minutes, followed by sterile filtration of the centrifuge supernatant through a 0.45μ type HA Millipore filter. Samples were then cryogenically preserved at -120°C until analysis could be run for soluble Chemical Oxygen Demand (C.O.D.) and U.V. scans performed. Ultra-frozen samples were quick thawed, mixed, then analyzed.

Assays for soluble COD were performed using the Hach Low level method (0-150mg/l) (Hach Chemicals).

U.V. scans were run on a Model 25 Beckman U.V./visible spectrophotometer with deionized water as the blank. Scans were run at 100 nm/min. with a chart speed of 2 in./min.; span of 1A switching to 2A when off scale. Wavelengths were scanned from 360 nm to 200 nm. Visible range (350 nm to 750 nm) scans were also conducted but showed no activity.

Radioisotope incorporation studies using the reactor

-51-

biomass for uptake measurements were conducted by the standard procedure of this laboratory. The effect of BNS on the uptake process was determined by adding increased amounts of BNS to the standard uptake mixture. All biomass samples were from Run #2.

Microscopic observations of the biomass appearance and population were conducted on a regular basis for evaluation of population.shifts or declines in the higher trophic segments of the sludge biomass. Photomicrographic records were made of these alterations.

Calculation of biomass solids reductions was based upon the average solids difference between the control and BNS reactor, at 0.5 - 2.0 ppm BNS, in Run #2. The experimental runs were conducted over three months in the field. Consequently, typical variations in the operational environment exerted its control over the experimentation by discontinuous temperature, influent strength and wastewater character.

Run #1 was carried out for about a 3 week period. Run #2, which picked up on lower concentrations of uncoupler not previously used, ran for a longer period of time.

-52-

## TABLE 18

Field Test - Run # 1
Uncoupler Operation Data

| Day # | BNS Dose (µg/L) | Biomass (µg/L) | COD (µg/L) |
|-------|-----------------|----------------|------------|
| 0     | 2.0             | 571            | 60         |
|       | Control (0)     | 497            | 35         |
| 3     | 4.0             | 920            | 75         |
|       | Control (0)     | 770            | 40         |
| 5     | 8.0             | 1,387          | 85         |
|       | Control (0)     | 1,104          | 38         |
| 7     | 8.0             | 1,582          | 103        |
|       | Control (0)     | 1,267          | 43         |
| 10    | 16.0            | 1,809          | 102        |
|       | Control (0)     | 1,117          | 45         |
| 12    | 16.0            | 1,688          | 101        |
|       | Control (0)     | 984            | 55         |
| 14    | 16.0            | 1,714          | 132        |
|       | Control (0)     | 926            | 35         |
| 17    | 32.0            | 1,516          | -          |
|       | Control (0)     | 760            | -          |
| 19    | 32.0            | 1,511          | $> 50$     |
|       | Control (0)     | 727            | 40         |

TABLE 18
(Cont'd)
Field Test - Run # 2

| Day # | BNS Dose (µg/L) | Biomass (µg/L) | COD (µg/L) |
|---|---|---|---|
| 0 | 0 | 556 | - |
| | Control (0) | 461 | - |
| 4 | 0.5 | 152 | 19 |
| | Control (0) | 386 | 40 |
| 6 | 0.5 | 354 | 25 |
| | Control (0) | 407 | 13 |
| 9 | 0.5 | 522 | 30 |
| | Control (0) | 448 | 30 |
| 11 | 1.0 | 472 | 30 |
| | Control (0) | 530 | 25 |
| 13 | 1.0 | 354 | 25 |
| | Control (0) | 413 | 12 |
| 16 | 2.0 | 301 | 20 |
| | Control (0) | 681 | 25 |
| 18 | 2.0 | 369 | 30 |
| | Control (0) | 454 | 60 |
| 20 | 2.0 | 527 | 55 |
| | Control (0) | 382 | 11 |
| 23 | 4.0 | 179 | 38 |
| | Control (0) | 529 | 21 |
| 25 | 4.0 | 907 | 45 |
| | Control (0) | 778 | 30 |
| 30 | 4.0 | 639 | >150 |
| | Control (0) | 881 | 125 |
| 34 | 4.0 | 514 | >150 |
| | Control (0) | 617 | 97 |
| 37 | 8.0 | 616 | >150 |
| | Control (0) | 597 | 45 |

## Conclusions Drawn From Field Evaluation

1)  Reduction in the biomass solids concentrations (18% average) were realized at relatively low doses of uncoupler (0.5 - 2.0 ppm BNS), without impairment of organic removal, when a field wastewater is employed as a substrate;

2)  Higher doses ($> 4$ ppm) of uncoupler cause changes in the sludge population, manifested by altered substrate uptake in the microorganisms; and

3)  Resistance of biomass to BNS ensues at higher uncoupler exposures, after dosages exceed 4 ppm.

While this invention has been described with respect to particular embodiments thereof, it is apparent that numerous other forms and modifications of this invention will be obvious to those skilled in the art. The appended claims and this invention generally should be construed to cover all such obvious forms and modifications which are within the true spirit and scope of the present invention.

. . . . . . . . . . . . . . . . . . . . . . . . . . . . . . . . . . . . . . . . . . . . . . . . . . . . . . . . .

CLAIMS

1. A method of increasing the capacity and rate of a microbial cell population's bioconversion of a substrate in an aqueous system, without an attendant significant increase in (i) the microbial cell population and (ii) solid waste production from said bioconversion, which microbial cells maintain their life cycle and reproduce through the production, use and storage of energy and the maintenance of an energy reservoir, characterized in that there is added to said system an effective amount of a substance or substances which will effectively impede the natural tendency of the microbial cell during its bioconversion of said substrate to attain its normal energy reservoir level and will thereby promote greater bioconversion of the substrate because of the microbial cell's attempt to achieve its normal energy reservoir.

2. A method according to claim 1, wherein said substance or substances uncouple(s) the microbial cells' energy production from substrate bioconversion by effectively blocking to the necessary degree energy transfer to the energy reservoir of the cells.

3. A method according to claim 1 or 2, wherein the amount of substance or substances used is such as to not totally block the transfer of the energy produced to the energy reservoir of the cells and thereby permit the cells to continue to live and bioconvert the substrate.

4. A method according to any of claims 1 to 3, wherein the substance or substances comprise an oxidative uncoupler or uncouplers.

5. A method according to claim 4, wherein the oxidative uncoupler is a nitrostyrene compound.

6. A method according to claim 5, wherein the nitrostyrene compound is beta-bromo-beta-nitrostyrene.

7. A method according to any of claims 1 to 3, wherein the substance or substances are selected from bis-(trichloromethyl)sulfone; 4, 7 diphenyl-1, 10 phenathroline; 2-mercaptobenzothiazole; myristic acid; N,N,N',N' tetramethyl p-phenylene diamine; rotenone; salicylanilide; tetraphenylboron; thiosalicyclic acid; dicyclohexylcarbodiimide; beta-bromo-beta-nitrostyrene; and mixtures thereof.

8. A method according to any of claims 1 to 7, wherein the substrate is an organic substrate.

9. A method according to claim 8, wherein the substrate is carbohydrate and the product produced is ethanol.

10. A method according to any of claims 1 to 7, wherein the substrate is an inorganic substrate.

11. A method according to any of claims 1 to 10, wherein the aqueous system is a waste water system.

12. A composition characterized by a member selected from

(A) beta-bromo-beta-nitrostyrene, and bis-(trichloromethyl)sulfone and at least one member selected from

(B) 4, 7 diphenyl-1, 10 phenathroline;
2-mercaptobenzothiazole;
myristic acid;
N,N,N',N' tetramethyl p-phenylene diamine;
rotenone;
salicylanilide;
tetraphenylboron; thiosalicylic acid; and
dicyclohexylcarbodiimide.